(19) Europäisches Patentamt
European Patent Office
Office européen des brevets

(11) Veröffentlichungsnummer: **0 252 431 B1**

(12) # EUROPÄISCHE PATENTSCHRIFT

(45) Veröffentlichungstag der Patentschrift: **13.03.91**

(51) Int. Cl.$^5$: **C07C 303/40**

(21) Anmeldenummer: **87109495.9**

(22) Anmeldetag: **02.07.87**

(54) **Verfahren zur Herstellung von N-Fluorsulfonamiden.**

(30) Priorität: **10.07.86 DE 3623184**

(43) Veröffentlichungstag der Anmeldung:
**13.01.88 Patentblatt 88/02**

(45) Bekanntmachung des Hinweises auf die Patenterteilung:
**13.03.91 Patentblatt 91/11**

(84) Benannte Vertragsstaaten:
**AT BE CH DE ES FR GB IT LI NL SE**

(56) Entgegenhaltungen:
**EP-A- 0 211 578**
**DE-A- 2 320 119**
**DE-A- 2 332 430**

(73) Patentinhaber: **Kali-Chemie Aktiengesellschaft**
**Postfach 220, Hans-Böckler-Allee 20**
**W-3000 Hannover 1(DE)**

(72) Erfinder: **Wilkes, Bernd**
**Eschenbachstrasse 1**
**W-3000 Hannover 61(DE)**
Erfinder: **Naumann, Dieter**
**Tunnelweg 9**
**W-4600 Dortmund 41(DE)**
Erfinder: **Rudolph, Werner**
**Oderstrasse 38**
**W-3000 Hannover 71(DE)**
Erfinder: **Sander, Rüdiger**
**Am Ladeholz 2**
**W-3163 Sehnde 1(DE)**

(74) Vertreter: **Lauer, Dieter, Dr.**
**c/o Kali-Chemie AG Postfach 220 Hans-Böckler-Allee 20**
**W-3000 Hannover 1(DE)**

EP 0 252 431 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von N-Fluorsulfonamiden.

Die Herstellung von N-Fluorsulfonamiden aus Sulfonamiden durch Umsetzung mit Fluorierungsmitteln in einem Lösungsmittel ist aus der DE-OS 23 32 430 an sich bekannt. Diese Verfahren sind aber einerseits durch Verwendung teurer Fluorierungsmittel wie z. B. Hypofluoriten unwirtschaftlich; bei Verwendung von Fluor als Fluorierungsmittel muß wegen der unselektiven Reaktion des Fluors bei sehr tiefen Temperaturen mit stark verdünntem Fluor gearbeitet werden, und selbst dann sind die Ausbeuten an Fluorsulfonamiden nicht befriedigend. So wird z. B. in JACS 1984 Seite 452 ff über die Herstellung von N-Fluor-N-Alkylsulfonamiden berichtet. Dort wird die Umsetzung mit 1 bis 5 % Fluor in Stickstoff bei -78 °C in $CFCl_3/CHCl_3$ als Lösungsmittel vorgenommen und dabei werden Ausbeuten im Bereich von 11 bis 71 % erreicht.

Din EP-A 211 578. ein älteres Recht. beschreibt die Herstellung bestimmter N-Fluorsulfonamide mit perfluorierten Alkylresten. Irgendein Hinweis auf die Verwendung Nitrile enthaltender Lösungsmittel bei der Fluorierung findet sich in diesem Dokument nicht.

Aufgabe der Erfindung ist es, die Nachteile bekannter Verfahren zu beheben und ein neues, verbessertes Verfahren zur Herstellung von N-Fluorsulfonamiden zur Verfügung zu stellen.

Das erfindungsgemäße Verfahren zur Herstellung von N-Fluorsulfonamiden geht aus vom gattungsgemäßen Verfahren, bei dem Sulfonamide mit Fluor in einem Lösungsmittel umgesetzt werden und ist dadurch gekennzeichnet daß man ein Sulfonamid der allgemeinen Formel I

$$R^1-SO_2-NH-R^2 \qquad (I)$$

in der

$R^1$ = Alkyl mit 1 bis 10 C-Atomen vorzugsweise 1 bis 8 C-Atomen, gegebenenfalls substituiert durch ein oder mehrere F-Atome oder
Aryl, wie Phenyl oder Naphthyl, gegebenenfalls substituiert durch 1 bis 5 F-Atome und/oder 1 bis 2 $CF_3$- oder $CH_3$-Gruppen und
$R^2$ = Alkyl mit 1 bis 6 C-Atomen

bedeutet, mit elementarem Fluor in einem Lösungsmittelgemisch aus einem Halogenkohlenwasserstoff und einem Nitril umsetzt und das entstehende N-Fluorsulfonamid aus dem Reaktionsgemisch isoliert.

Als Substituenten $R^1$ kommen bevorzugt in Frage

a) für den Fall Alkyl Substituenten Perfluoralkyl wie z.B. Perfluoroctyl, Pentafluoräthyl, Trifluormethyl, insbesondere Trifluormethyl,

b) für den Fall Aryl die Bedeutung Tolyl, Trifluormethylphenyl, Fluorphenyl, Perfluorphenyl besitzt.

Als Substituent $R^2$ kommt geradliniges oder verzweigtes Alkyl in Frage.

Unverzweigtes Alkyl ist dabei insbesondere Methyl, Äthyl, Propyl, Butyl, Pentyl, Hexyl.

Verzweigtes Alkyl ist insbesondere iso-Propyl, iso-Butyl, sec-Butyl, tert-Butyl, 1-,2-, oder 3-Methyl-butyl, 2-oder 3-Äthyl-Propyl, 1,1-,2,2-3,3-Dimethylpropan. Besonders bevorzugtes verzweigtes Alkyl sind iso-Propyl, iso-Butyl, sec-Butyl, neo-Pentyl (2,2-Dimethylpropyl). Besonders bevorzugtes verzweigtes Alkyl ist jenes, dessen Verzweigung nicht am mit dem Stickstoff verbundenen C-Atom beginnt. Solch verzweigtes Alkyl ist beispielsweise i-Butyl, sec-Butyl, neo-Pentyl.

Als Bestandteil des Lösungsmittelgemisches wird bevorzugt ein Halogenkohlenwasserstoff aus der Gruppe Chlorkohlenwasserstoffe und/oder Fluorchlorkohlenwasserstoffe mit 1 bis 3 C-Atomen ausgewählt. Besonders bewährt als Halogenkohlenwasserstoff haben sich Dichlormethan, Chloroform, Tetrachlorkohlenstoff, Fluortrichlormethan, Trifluor-Trichloräthan, Tetrafluor-Dichloräthan. Bei Halogenkohlenwasserstoffen mit 2 oder 3 C-Atomen, die Isomere bilden, können alle bekannten Isomere zur Anwendung gelangen, wobei allerdings die asymmetrisch substituierten Isomere bevorzugt werden. So kommt beispielsweise als Trifluortrichloräthan außer dem 1.1.1-Trifluortrichloräthan bevorzugt das 1.1.2-Trifluortrichloräthan als Lösungsmittelbestandteil in Frage.

Als weiterer, zwingender Bestandteil des Lösungsmittelgemisches wird ein Nitril eingesetzt, wobei Acetonitril und/oder Propionitril, insbesondere Acetonitril bevorzugt werden.

Das Mischungsverhältnis zwischen Halogenkohlenwasserstoff und Nitril kann in recht weitem Verhältnis variiert werden. Bewährt haben sich volumenmäßige Mischungsverhältnisse Halogenkohlenwasserstoff:Nitril im Bereich von 10:1 bis 1:10, vorzugsweise 2:1 bis 1:2.

Das Fluorierungsmittel Fluor wird bevorzugt im Gemisch mit Inertgasen wie Stickstoff, $SF_6$, $CF_4$ oder Edelgasen wie Helium, Neon, Argon, Krypton eingesetzt. Bevorzugtes Inertgas ist Stickstoff. Für die Fluorierung können Fluor/Inertgas-Gemische eingesetzt werden, die bis zu 30 Vol.-% Fluor enthalten. Sehr gute Ergebnisse wurden mit 10 bis 25 Vol.-% Fluor erhalten.

Die Temperatur bei der die Umsetzung durchgeführt wird, kann in weiten Bereichen variiert werden und liegt insbesondere im Bereich von +10 bis -80 °C. Die Wahl der Temperatur hängt im Einzelfall von der Wahl der Reaktionsbedingungen wie Fluorkonzentration, Zusammensetzung des Lösungsmittelgemisches etc. ab.

Eine weitere vorteilhafte Ausgestaltung der Erfindung sieht vor, das Reaktionsgemisch während und/oder nach der Umsetzung mit einem Adsorptionsmittel für Fluorwasserstoff in Kontakt zu bringen und auf diese Art im Verlauf der Umsetzung gebildeten Fluorwasserstoff zu entfernen. Als Adsorptionsmittel für Fluorwasserstoff kommen alle an sich bekannten Adsorptionsmittel in Frage, die nicht störend in die Reaktion eingreifen bzw. sich nicht mit dem gebildeten N-Fluorsulfonamid umsetzen. Als besonders gut geeignete Adsorptionsmittel haben sich Alkalifluoride, -hydrogencarbonate, -carbonate, insbesondere Natriumfluorid bewährt.

Die Aufarbeitung des Reaktionsgemisches kann in an sich bekannter Weise durch chromatographische Methoden, z.B. Säulenchromatographie erfolgen. Es ist aber auch möglich, das Reaktionsgemisch destillativ aufzuarbeiten. Diese Möglichkeit ist vor allem dann erfolgreich anwendbar, wenn aus dem resultierenden Reaktionsgemisch der Fluorwasserstoff sorgfältig entfernt wird.

In einer besonderen Variante sieht die Erfindung vor, das Verfahren kontinuierlich zu führen, indem man

a) in einen thermostatisierten Reaktor kontinuierlich eine Lösung eines Sulfonamids entsprechend Formel (I) in einem Lösungsmittelgemisch aus Halogenkohlenwasserstoff und Nitril sowie gasförmiges Fluor eindosiert,

b) das Reaktionsgemisch im Kreislauf über ein Adsorptionsmittel für Fluorwasserstoff führt,

c) aus dem Reaktor und/oder aus dem Kreislauf gemäß Stufe b) kontinuierlich und entsprechend der Zugabe an Reaktionskomponenten Reaktionsgemisch entnimmt,

d) aus dem entnommenem Reaktionsgemisch gegebenenfalls noch enthaltenen Fluorwasserstoff mit einem Adsorptionsmittel entfernt und

e) das N-Fluorsulfonamid gewinnt, vorzugsweise durch chromatographische Verfahren oder durch Destillation.

Das erfindungsgemäße Verfahren zeichnet sich gegenüber bekannten Verfahren durch erhebliche Vorteile aus. So ist es möglich, die Reaktionsprodukte in stark verbesserten Ausbeuten zu erhalten. Ferner konnten praktisch keine Nebenprodukte nachgewiesen werden; d.h. die Reaktion läuft bei erfindungsgemäßer Verfahrensführung mit sehr hoher Selektivität ab.

Gemäß dem erfindungsgemäßen Verfahren hergestollten sind insbesondere N-Fluorsulfonamide der Formel (II)

$$R^3-SO_2-NF-R^2 \qquad (II)$$

in der

$R^3$ ein 1 oder mehrere F-Atome enthaltender Rest aus der Gruppe Phenyl, Tolyl oder Alkyl mit 1 bis 10 C-Atomen, vorzugsweise 1 bis 8 C-Atomen und

$R^2$ ein Alkylrest mit 1 bis 6 C-Atomen bedeutet.

Von diesen Verbindungen seien insbesondere jene angeführt, in denen die Substituenten $R^3$ und $R^2$ jeweils die in Tabelle 1 angegebene Bedeutung besitzen.

Tabelle 1 Verbindungen der Formel (II)

| $R^3$ | $R^2$ |
|---|---|
| $CF_3$ | $CH_3$, $C_2H_5$, n-$C_3H_7$, n-$C_4H_9$, sec-$C_4H_9$<br>iso-$C_4H_9$, n-$C_5H_{11}$, neo-$C_5H_{11}$, n-$C_6H_{13}$ |
| $C_2F_5$ | $CH_3$, $C_2H_5$, n-$C_3H_7$, n-$C_4H_9$, sec-$C_4H_9$<br>iso-$C_4H_9$, n-$C_5H_{11}$, neo-$C_5H_{11}$, n-$C_6H_{13}$ |
| $C_8F_{17}$ | $CH_3$, $C_2H_5$, n-$C_3H_7$, n-$C_4H_9$, sec-$C_4H_9$<br>iso-$C_4H_9$, n-$C_5H_{11}$, neo-$C_5H_{11}$, n-$C_6H_{13}$ |
| $C_6H_4F$ (para F) | $C_2H_5$, n-$C_3H_7$, n-$C_4H_9$, sec-$C_4H_9$<br>iso-$C_4H_9$, n-$C_5H_{11}$, neo-$C_5H_{11}$, n-$C_6H_{13}$ |
| $C_6H_4CF_3$ (para $CF_3$) | $CH_3$, $C_2H_5$, n-$C_3H_7$, n-$C_4H_9$, sec-$C_4H_9$<br>iso-$C_4H_9$, n-$C_5H_{11}$, neo-$C_5H_{11}$, n-$C_6H_{13}$ |
| $C_6F_5$ | $CH_3$, $C_2H_5$, n-$C_3H_7$, n-$C_4H_9$, sec-$C_4H_9$<br>iso-$C_4H_9$, n-$C_5H_{11}$, neo-$C_5H_{11}$, n-$C_6H_{13}$ |

Die folgenden Beispiele sollen die Erfindung näher charakterisieren ohne sie in ihrem Umfange zu begrenzen. Beispiele 1 und 3 sind erfindungungsgemäße Beispiele; Beispiel 2 ist ein Vergleichsbeispiel.

Beispiel 1

50 g (207 m Mol) p-Toluol-N-neopentylsulfonamid werden in 300 ml einer Mischung aus $CH_3CN$ und $CCl_3F$ im Verhältnis (Vol.) 1:1 gelöst und in einem temperierbaren Reaktor auf -30° C gekühlt.

Zur besseren Durchmischung wird die Reaktionslösung mittels einer HF- und Fluor-beständigen Pumpe umgepumpt. Das Fluor-Stickstoff-Gasgemisch ( 15 Vol.-% $F_2$ 85 % $N_2$) wird dann in die Lösung eingeleitet. Nach Erreichen von ca. 95 % Umsatzgrad (Kontrolle durch Dünnschichtchromatographie) wird die Fluorzufuhr unterbrochen und die Lösung fluorfrei gespült. Zum Entfernen des HF wird die Reaktionslösung über NaF-Pellets filtriert. Die weitere Aufarbeitung erfolgt durch Abziehen des Lösemittels im Vakuum und säulenchromatographische Reinigung ($SiO_2$, $CH_2Cl_2$) des öligen Rohproduktes. Nicht umgesetztes Ausgangsm

**Ausbeute: 36,5 g ≙ 76 %** **Selektivität 98-99 %**

**Reinheit: > 97 %**

aterial wird zurückgeführt.

überraschenderweise kann das bei 58 °C schmelzende Produkt unzersetzt im Hochvakuum destilliert werden.

Kp: 135 °C / 3 $10^{-3}$ Torr.
δ $^{19}F$-NMR: -38 ppm
$J^3$ ($^{19}F$-$^1H$): 46 Hz

Beispiel 2

Die Fluorierung von p-Toluol-N-neopentylsulfonamid wurde gemäß Beispiel 1, jedoch in einer 1:1 Mischung aus $CH_2Cl_2$ und $CCl_3F$ durchgeführt. Nach gleicher Reaktionszeit wie im Beispiel 1 wurde die Fluorzufuhr beendet und die Reaktionsmischung aufgearbeitet.

Isoliert wurden: 2,4 g Produkt
2,1 g Tosylfluorid (Nebenprodukt)
14,0 g nicht umgesetztes Edukt.

Weitere Zersetzungsprodukte wurden nicht isoliert. Der Umsatzgrad bei dieser Reaktionsführung beträgt somit 30 %, die Selektivität 53.3 %.

Beispiel 3

a) Die Fluorierung von p-Toluol-N-methylsulfonamid (5 g ≙ 27 m Mol) erfolgte in einem temperierbaren Reaktor bei -30 °C und mit einer Fluorkonzentration von 15 Vol.-% (in $N_2$). Als Lösemittel wurde eine 2:1 Mischung aus $CCl_3F$ und $CH_3CN$ eingesetzt.

Nach Aufarbeitung gemäß Beispiel 1 konnten 4,3 g Produkt (21,2 m Mol) isoliert werden, 78,5 % Ausbeute. Die Destillation des bei 47 °C schmelzenden Feststoffes erfolgt bei 90 °C und 3 $10^{-2}$ Torr.
δ $^{19}F$-NMR : -37 ppm
$J^3$ ($^{19}F$-$^1H$): 32 Hz.

Entsprechend wurden die in der folgenden Tabelle charakterisierten Verbindungen erhalten:

| $R^1$ bzw. $R^3$ | $R^2$ | δ $^{19}F$(ppm)(N-F) | $^3J$ ($^{19}F$-$^{19}F$)(Hz) |
|---|---|---|---|
| p-Tolyl | n-Butyl | - 50,3 | 41,0 t |
| p-Tolyl | n-Propyl | - 50,3 | 41,2 t |
| $CF_3$ | $CH_3$ | - 40,68 | 6,9 d (F-F) |
| | | | ∼ 30 m (F-H) |
| $CF_3$ | n-Butyl | - 40,0 | d (F-F) |
| | | | m (F-H) |
| $C_6F_5$ | n-Propyl | - 50,2 | 40,05 t |
| $C_6F_5$ | neo-Pentyl | - 36,6 | 40,05 t |
| tert-Butyl | $CH_3$ | - 39,76 | 34,33 q |
| tert-Butyl | neo-Pentyl | - 52,7 | 40,05 t |
| tert-Butyl | n-Butyl | - 52,7 | 42,3 t |

Standard: $CCl_3F$, Meßfrequenz 75,26 M/Hz.

## Ansprüche

1. Verfahren zur Herstellung von N-Fluorsulfonamiden durch Umsetzung von Sulfonamiden mit Fluor in einem Lösungsmittel, dadurch gekennzeichnet, daß man ein Sulfonamid der allgemeinen Formel I

$$R^1-SO_2-NH-R^2 \qquad (I)$$

in der

$R^1$ = Alkyl mit 1 bis 10 C-Atomen, gegebenenfalls substituiert durch ein oder mehrere F-Atome oder
Aryl gegebenenfalls substituiert durch 1 bis 5 F-Atome und/oder 1 bis 2 $CF_3$ -oder $CH_3$-Gruppen und
$R^2$ = Alkyl mit 1 bis 6 C-Atomen

bedeutet, mit elementarem Fluor in einem Lösungsmittelgemisch aus einem Halogenkohlenwasserstoff und einem Nitril umsetzt und das entstehende N-Fluorsulfonamid aus dem Reaktionsgemisch isoliert.

2. Verfahren gemäß Anspruch 1, dadurch gekennzeichnet, daß $R^1$
a) für den Fall Alkyl die Bedeutung Perfluoralkyl, insbesondere Trifluormethyl,
b) für den Fall Aryl die Bedeutung Tolyl, Trifluormethylphenyl, Fluorphenyl, Perfluorphenyl besitzt.

3. Verfahren nach einem der vorhergehenden Ansprüche, dadurch gekennzeichnet, daß man einem Halogenkohlenwasserstoff aus der Gruppe Chlorkohlenwasserstoffe und/oder Fluorchlorkohlenwasserstoffe mit 1 bis 3 C-Atomen als Lösungsmittel-Bestandteil einsetzt.

4. Verfahren nach Anspruch 3, dadurch gekennzeichnet daß man als Halogenkohlenwasserstoff Dichlormethan, Chloroform, Tetrachlorkohlenstoff, Fluortrichlormethan, Trifluor-Trichloräthan, Tetrafluor-Dichloräthan einsetzt.

5. Verfahren nach einem der vorhergehenden Ansprüche, dadurch gekennzeichnet, daß man als Nitril Acetonitril und/oder Propionitril einsetzt.

6. Verfahren nach einem der vorhergehenden Ansprüche, dadurch gekennzeichnet, daß man Halogenkohlenwasserstoff und Nitril in einem Volumenverhältnis von 10:1 bis 1:10, vorzugsweise 2:1 bis 1:2 einsetzt.

7. Verfahren nach einem der vorhergehenden Ansprüche, dadurch gekennzeichnet, daß man Fluor im Gemisch mit einem Inertgas einsetzt, wobei das Gemisch bis zu 30 Vol.-%, vorzugsweise 10 bis 25 Vol.-% Fluor enthält.

8. Verfahren nach einem der vorhergehenden Ansprüche, dadurch gekennzeichnet, daß man das Reaktionsgemisch während und/oder nach der Umsetzung mit einem Adsorptionsmittel für Fluorwasserstoff in Kontakt bringt.

9. Kontinuierliches Verfahren zur Herstellung von N-Fluorsulfonamiden gemäß einem der vorhergehenden Ansprüche, dadurch gekennzeichnet. daß man
a) in einen thermostatisierten Reaktor kontinuierlich eine Lösung eines Sulfonamids entsprechend Formel (I) in einem Lösungsmittelgemisch aus Halogenkohlenwasserstoff und Nitril sowie gasförmiges Fluor eindosiert.
b) das Reaktionsgemisch im Kreislauf über ein Adsorptionsmittel für Fluorwasserstoff führt,
c) aus dem Reaktor und/oder aus dem Kreislauf gemäß Stufe b) kontinuierlich und entsprechend der Zugabe an Reaktionskomponenten Reaktionsgemisch entnimmt,
d) aus dem entnommenem Reaktionsgemisch gegebenenfalls noch enthaltenen Fluorwasserstoff mit einem Adsorptionsmittel entfernt und
e) das N-Fluorsulfonamid gewinnt, vorzugsweise durch chromatographische Verfahren oder durch Destillation.

## Claims

1. Process for the preparation of N-fluorosulphonamides by reacting sulphonamides with fluorine in a solvent, characterized in that a sulphonamide of the general Formula I

$$R^1\text{-}SO_2\text{-}NH\text{-}R^2 \qquad (I)$$

in which

$R^1$ = alkyl with 1 to 10 C atoms, optionally substituted by one or more F atoms or aryl optionally substituted by 1 to 5 F atoms and/or 1 to 2 $CF_3$ or $CH_3$ groups and

$R^2$ = alkyl with 1 to 6 C atoms,

is reacted with elemental fluorine in a solvent mixture consisting of a halogenated hydrocarbon and a nitrile and the resulting N-fluorosulphonamide is isolated from the reaction mixture.

2. Process according to Claim 1, characterized in that $R^1$
   a) in the case of alkyl has the meaning perfluoroalkyl, in particular trifluoromethyl,
   b) in the case of aryl has the meaning tolyl, trifluoromethylphenyl, fluorophenyl or perfluorophenyl.

3. Process according to one of the preceding Claims, characterized in that a halogenated hydrocarbon from the group chlorohydrocarbons and/or fluorochlorohydrocarbons with 1 to 3 C atoms is used as a constituent of the solvent.

4. Process according to Claim 3, characterized in that dichloromethane, chloroform, carbon tetrachloride, fluorotrichloromethane, trifluoro-trichloroethane or tetrafluoro-dichloroethane is used as the halogenated hydrocarbon.

5. Process according to one of the preceding Claims, characterized in that acetonitrile and/or propionitrile is used as the nitrile.

6. Process according to one of the preceding Claims, characterized in that halogenated hydrocarbon and nitrile are used in a volume ratio of 10:1 to 1:10, preferably 2:1 to 1:2.

7. Process according to one of the preceding Claims, characterized in that fluorine is used in a mixture with an inert gas, with the mixture containing up to 30% by volume, preferably 10 to 25% by volume, fluorine.

8. Process according to one of the preceding Claims, characterized in that the reaction mixture is brought into contact with an adzorption agent for hydrogen fluoride during and/or after the reaction.

9. Continuous process for the preparation of N-fluorosulphonamides according to one of the preceding Claims, characterized in that
   a) a solution of a sulphonamide corresponding to Formula (I) in a solvent mixture of halogenated hydrocarbon and nitrile, and also gaseous fluorine, are metered continuously into a thermostatic reactor,
   b) the reaction mixture is recirculated via an adsorption agent for hydrogen fluoride,
   c) reaction mixture is removed from the reactor and/or from the circuit in stage b) continuously and corresponding to the addition of reaction constituents,
   d) optionally any hydrogen fluoride still contained is removed from the reaction mixture which has been removed using an adsorption agent and
   e) the N-fluorosulphonamide is obtained, preferably by chromatographic processes or by distillation.

## Revendications

1. Procédé de préparation de N-fluorosulfamides, par réaction de sulfamides avec du fluor dans un solvant, caractérisé en ce qu'on fait réagir un sulfamide de formule générale I

$$R^1 - SO_2 - NH - R^2 \qquad (I)$$

[dans laquelle

$R^1$ représente un groupe alkyle ayant 1 à 10 atomes de carbone, éventuellement substitué par un ou plusieurs atomes de F, ou

un groupe aryle eventuellement substitué par 1 à 5 acomes de F et/ou 1 à 2 groupes $CF_3$ ou $CH_3$,

$R^2$ représente un groupe alkyle ayant 1 à 6 atomes de carbone] avec du fluor élémentaire dans un mélange de solvants,formé par un hydrocarbure halogéné et un nitrile, et l'on isole du mélange réactionnel le N-fluorosulfamide résultant.

2. Procédé selon la revendication 1 , caractérisé en ce que:
a) dans le cas d'un groupe alkyle, $R^1$ représente un groupe perfluoralkyle , en particulier trifluorométhyle ,

b) dans le cas d'un groupe aryle , $R^1$ représente un groupe tolyle , trifluorométhylphényle , fluorophényle ,perfluorophényle.

3. Procédé selon l'une des revendications précédentes , caractérisé en ce qu'on utilise,comme composant du mélange des solvants , un hydrocarbure halogéné,choisi parmi les hydrocarbures chlorés et/ou les hydrocarbures fluorés -chlorés ayant 1 à 3 atomes de carbone.

4. Procédé selon la revendication 3 , caractérisé en ce qu'on utilise comme hydrocarbure halogéné le dichlorométhane , le chloroforme , le tétrachlorure de carbone , le fluorotrichlorométhane , le trifluorotrichloréthane , le tétrafluoro-dichloréthane .

5. Procédé selon l'une des revendications précédentes , caractérisé en ce qu'on utilise l'acétonitrile et/ou le propionitrile comme nitrile .

6. Procédé selon l'une des revendications précédentes , caractérisé en ce qu'on utilise l'hydrocarbure halogéné et le nitrile selon un rapport en volumes de 10:1 à 1:10,avantageusement de 2:1 à 1:2.

7. Procédé selon l'une des revendications précédentes , caractérisé en ce qu'on utilise le fluor en mélange avec un gaz inerte , le mélange contenant jusqu'à 30 % en volume , avantageusement 10 à 25 % en volume de fluor . 8. Procédé selon l'une des revendications précédentes , caractérisé en ce que,pendant et/ou après la réaction, on met le mélange réactionnel en contact avec un agent d'adsorption de l'acide fluorhydrique.

8. Procédé continu de préparation de N-fluorosulfamides selon l'une des revendications précédentes , caractérisé en ce que :
(a) dans un réacteur thermostaté,on introduit en continu,de façon dosée , une solution d'un sulfamide répondant à la formule (I),dans un mélange de solvants formé par un hydrocarbure halogéné et un nitrile , ainsi que du fluor gazeux ;
(b) on fait passer en circuit le mélange réactionnel sur un agent d'adsorption de l'acide fluorhydrique ;
(c) on prélève en continu,et selon l'addition de constituants destinés à la reaction, le mélange réactionnel du réacteur et/ou du circuit en boucle selon l'étape (b) ;
(d) on élimine, à l'aide d'un agent d'adsorption , l'acide fluorhydrique éventuellement encore contenu dans le mélange réactionnel prélevé ; et
(e) on récupère le N-fluorosulfamide, avantageusement par un procédé chromatographique ou par distillation .